# EUROPEAN PATENT SPECIFICATION

(11) **EP 2 326 368 B1**
(45) Date of publication and mention of the grant of the patent: **14.03.2018**
(21) Application number: 09766103.7
(22) Date of filing: 10.06.2009
(51) Int. Cl.: A61M 5/20, A61J 1/20, A61M 5/32

(54) **AUTOMATIC INJECTION DEVICE WITH TRIGGER LOCK**
AUTOMATISCHE INJEKTIONSVORRICHTUNG MIT AUSLÖSESPERRE
DISPOSITIF D'INJECTION AUTOMATIQUE À VERROU DE SÉCURITÉ

(30) Priority: 19.06.2008 GB 0811348
(43) Date of publication of application: 01.06.2011
(60) Divisional application: 13177327.7
(73) Proprietor: Cilag GmbH International, 6300 Zug (CH)
(72) Inventor: JENNINGS, Douglas, Ivan, Melbourn Hertfordshire SG8 6DP (GB); BURNELL, Rosemary, Louise, Melbourn Hertfordshire SG8 6DP (GB); HOGWOOD, Jonathan, Melbourn Hertfordshire SG8 6DP (GB)
(74) Representative: Brunner, John Michael Owen
(86) International application number: PCT/GB2009/001447
(87) International publication number: WO 2009/153542

(56) References cited:
- WO-A1-2006/106294
- WO-A1-2007/036676
- WO-A1-2007/051330
- US-A1- 2003 105 430
- US-A1- 2006 270 986

## Description

### Field of the Invention

The present invention relates to an injection device of the type that receives a syringe, extends the syringe and discharges its contents, commonly known as an auto-injector.

### Background of the Invention

Auto-injectors are known from WO 95/35126 and EP-A-0 516 473 and tend to employ a drive spring and some form of release mechanism that releases the syringe from the influence of the drive spring once its contents are supposed to have been discharged, to allow it to be retracted by a return spring.

An auto-injector is known from WO 2007/036676 which has a locking mechanism which must be disengaged before the release mechanism can be activated. In its locked position, the locking mechanism also prevents forward movement of the syringe out of the injection device against the bias of the return spring, for example when a cap gripping a boot covering the syringe needle, is removed. In the injection device described in WO 2007/036676, the locking mechanism comprises a sleeve which protrudes from an open end of the injection device. The sleeve is biased into its extended position by a resilient spring mechanism which must be overcome to disengage the locking mechanism. The locking mechanism can be disengaged by, for example, moving the sliding sleeve inwardly into the injection device. This can be done by forcing the end of the sliding sleeve against tissue and then activating the release mechanism.

The sleeve is surrounded by the housing of the injection device which causes friction to act against movement of the sliding sleeve. This is undesirable because it requires a certain amount of force acting on the injection device to be applied against tissue which can be painful to a user and give the feeling that the device is not operating adequately. Moreover, friction can prevent the sleeve moving back out of the injection device because the resilient spring mechanism may not be sufficient to overcome the friction between the housing and the sleeve. Furthermore, the rim of the sleeve which, in the engaged position of the locking mechanism, protrudes from the end of the housing, may catch on the rim of the housing which surrounds the sleeve, thereby preventing the sleeve from automatically returning to its engaged position, for example if the injection device is removed away from tissue before activation of the release mechanism.

Having the locking mechanism freely disengaged is undesirable because the release mechanism can be activated unintentionally causing accidental activation of the injection device. This is both dangerous and wasteful.

US 2003/105430 A1 discloses an injector where the needle is injected automatically into the injection site (e.g. a patient's skin). Delivery is initiated upon activation of the injector, and the needle is retracted after the end of delivery. Prior to and after injection, the needle is withdrawn into the device so as to avoid any potential injury or health risk to the user or health care provider.

WO 2007/051330 A1 discloses an injection device, especially an automatic injector.

US 2006/270986 A1 discloses a device for preparing an injection apparatus for making an injection.

### Summary of the Invention

The injection device of the present invention is designed to deal with the aforementioned problems.

In one aspect of the invention, there is provided an injection device comprising:
a housing adapted to receive a syringe having a discharge nozzle, the syringe being moveable in the housing on actuation of the injection device along a longitudinal axis from a retracted position in which the discharge nozzle is contained within the housing and an extended position in which the discharge nozzle of the syringe extends from the housing through an exit aperture;
an actuator;
a drive adapted to be acted upon by the actuator and in turn act upon the syringe to advance it from its retracted position to its extended position and discharge its contents through the discharge nozzle;
a locking mechanism moveable in a direction into the housing at the exit aperture from an engaged position into a disengaged position,
wherein the locking mechanism is adapted to prevent actuation of the device when it is in its engaged position and permit actuation of the device when it is in its disengaged position,
wherein the exit aperture is defined by a rim located on an edge of the housing, and
wherein the locking mechanism comprises a contact surface which is adapted to extend over or around at least a part of the rim.

By providing a contact surface, for example in the form of a flange, the locking mechanism can be more easily engaged and disengaged. This is because the contact surface provides an improved contact area against tissue. This means that point pressure from the locking mechanism applied to tissue is reduced. Moreover, the contact surface prevents the locking mechanism becoming caught, by friction or snagging, on the rim of the exit aperture. Thus, safer use of the injection device is achieved. According to the invention, the locking mechanism further comprises at least one arm extending from the contact surface into the housing. The arm may extend into the housing through the exit aperture. The arm does not contact the entire circumference on the internal housing surface of the rim. Thus, there is reduced friction between the rim/housing and the arm (when compared to a simple straight sleeve arrangement). Hence, the locking mechanism is less likely to become caught or snag.

Preferably, the rim of the exit aperture is elliptical or circular and the arm comprises an elliptical or circular cross-section shaped and positioned in such a way that it resides, in part, within an inner surface of the exit aperture.

Alternatively, the rim comprises an aperture through which the arm extends into the housing. The aperture supports the arm and imparts structural strength to the locking mechanism.

The locking mechanism may comprise a plurality of arms. More preferably, the locking mechanism comprises a pair of arms.

In an alterative embodiment of the invention, the locking mechanism may further comprise a sleeve extending from the contact surface into the housing. Preferably, the sleeve is dimensioned to fit within the exit aperture.

The rim of the exit aperture may be elliptical or circular and the sleeve may then comprise an elliptical or circular cross-section shaped and positioned in such a way that it fits within an inner surface of the exit aperture.

Preferably, the contact surface is formed on a first side of a hoop, and a second side of the hoop opposite the first side faces the rim of the exit aperture. In this arrangement, the second side moves towards the rim when the locking mechanism is moved from its engaged position to its disengaged position. Preferably, the release mechanism is arranged on the injection device, such that an inner radius of the hoop surrounds the rim when the release mechanism is in its disengaged position.

In one embodiment of the present invention, the injection device, further comprises:
a syringe carrier for carrying the syringe as it is advanced and restraining its advancement beyond its extended position, wherein the syringe carrier is adapted to support the syringe;
a latch member adapted to prevent, in an engaged position of the locking mechanism, movement of the syringe carrier relative to the housing and further adapted to permit, in a disengaged position of the locking mechanism, the syringe carrier moving relative to the housing.

Preferably, the locking mechanism comprises a primary member movable between the engaged position and the disengaged position.

The primary member may be the arm connected to the contact surface. Alternatively, the primary member may be the sleeve connected to the contact surface.

The primary member may include a latch opening through which the latch member projects before it engages a locking surface on the syringe carrier, the primary member acting as a cam and the latch member as a cam follower, so that movement of the primary member from its engaged position to its disengaged position causes the latch member to disengage from the locking surface.

The latch member may include a ramped surface against which a surface of the primary member acts to disengage it from the locking surface. Advantageously, the latch member may be provided on the housing.

In one embodiment of the invention, the injection device comprises a release mechanism which is moveable between an unactuated position and an actuated position,
wherein the release mechanism is adapted to prevent the actuator acting on the drive when in its unactuated position and permits the actuator to act on the drive when in its actuated position. Preferably, wherein the locking mechanism further comprises an interlock member movable between a locking position when the locking mechanism is in its engaged position, at which it prevents movement of the release mechanism from its unactuated position to its actuated position, and a releasing position when the release mechanism is in its disengaged position, at which it allows movement of the release mechanism from its unactuated position to its actuated position.

### Brief Description of the Drawings

The present invention is now described by way of example with reference to the accompanying drawings, in which:-
Fig. 1 is a perspective end view of one end of injection device according to one embodiment of the invention before a cap is affixed to it;
Fig. 2 is a perspective end view of the injection device according to Fig. 1 once the cap has been affixed;
Fig. 3 is a side cross-sectional view of the injection device of Fig. 1;
Figs. 4a and 4b are top cross-sectional views of the injection device of Fig. 1;
Fig. 5 is an enlarged cut-out from Fig. 4b;
Fig. 6 is a sectional schematic how an injection device may be further modified;
Fig. 7 is a cut-away view of such a modified injection device; and
Figs. 8a and 8b show an end of injection device according to an alternative embodiment of the invention

### Detailed Description of the Drawings

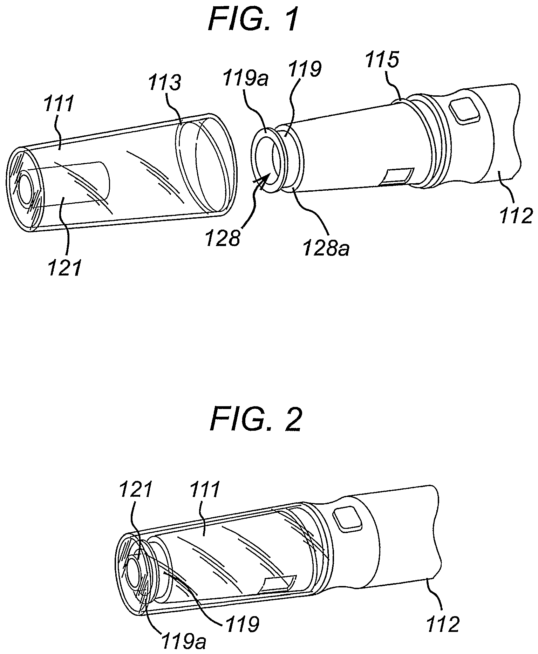

Fig. 1 shows the end of an injection device housing 112 and a cap 111. Other parts of the device will be described in greater detail below, but it will be seen that the cap 111 includes a thread 113 that cooperates with a corresponding thread 115 on the end of the housing. The end of the housing 112 has an exit aperture 128 (formed by rim 128a), from which the end of a sleeve 119 can be seen to emerge. The cap 111 has a central boss 121 that fits within the sleeve 119 when the cap 111 is installed on the housing 112, as can be seen in Fig. 2.

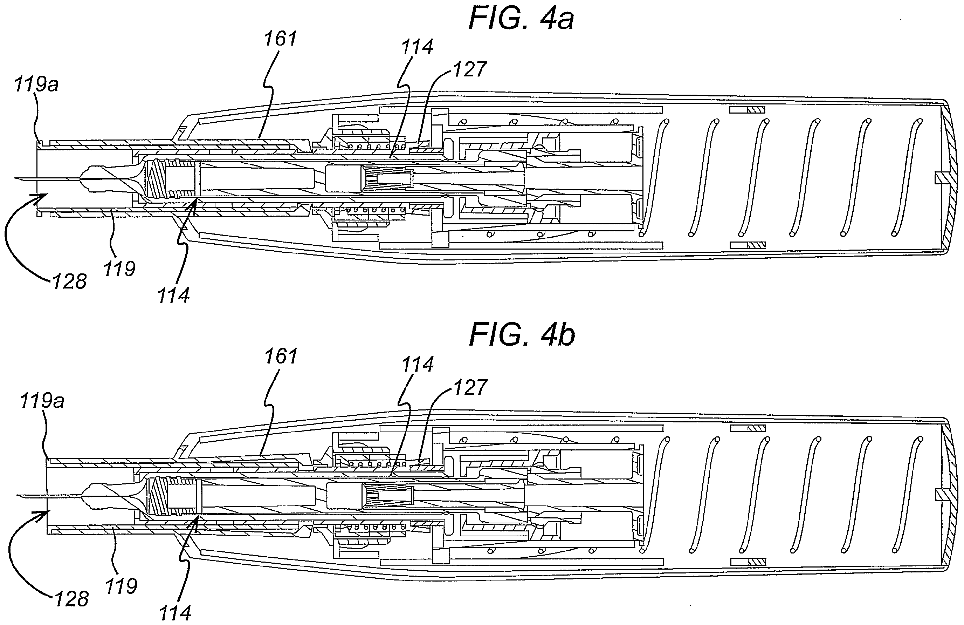

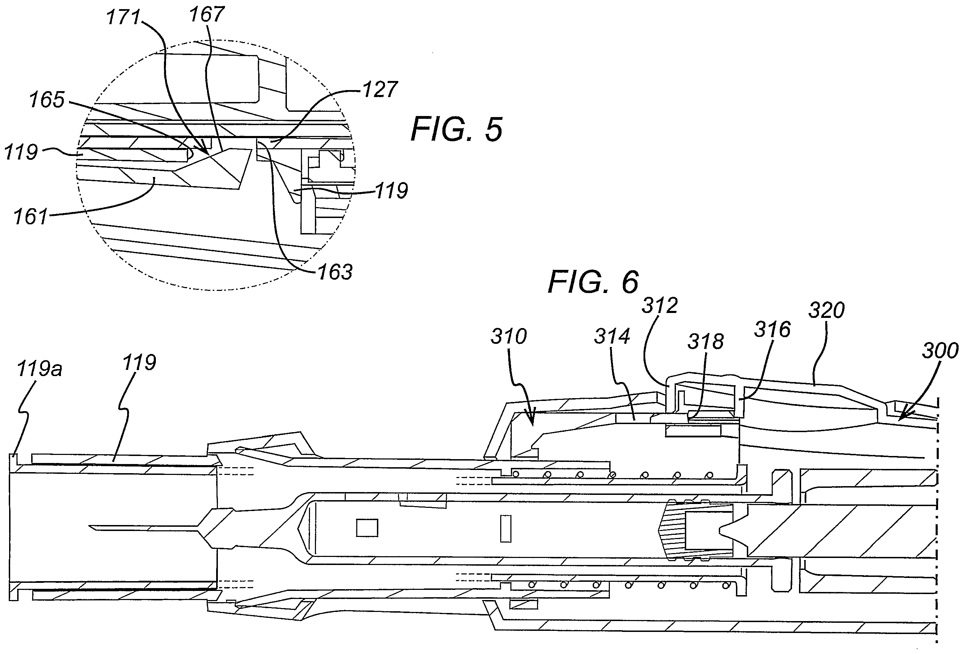

The sleeve 119 has a flange 119a on its exposed end having a contact surface 119b which is adapted to contact tissue when pressed against it. The sleeve 119 can slide from a locked position in which the flange 119a is spaced from the rim 128a, to an unlocked position in which the flange 119a has been pushed into a position in which it sits adjacent, in contacting juxtaposition, to the rim 128a. This is shown and explained in more detail in connection with Figs. 4a, 4b and 5 below.

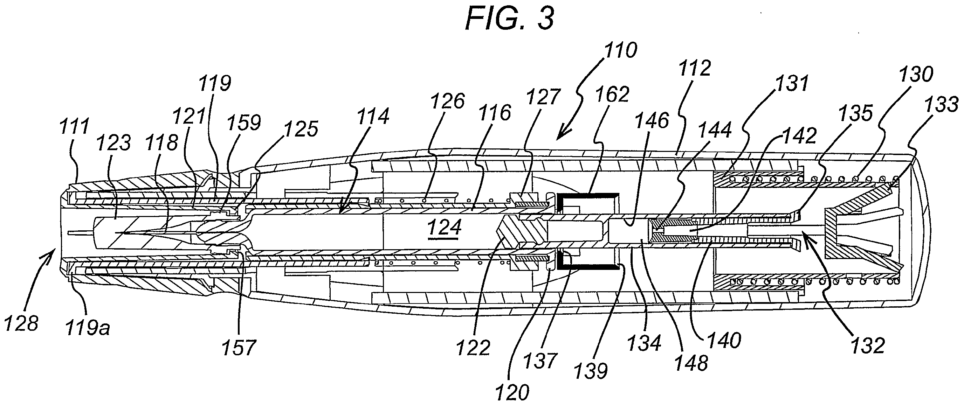

Fig. 3 shows an injection device 110 in more detail. The housing 112 contains a hypodermic syringe 114 of conventional type, including a syringe body 116 terminating at one end in a hypodermic needle 118 and at the other in a flange 120. The conventional plunger that would normally be used to discharge the contents of the syringe 114 manually has been removed and replaced with a drive element 134 that terminates in a bung 122. The bung 122 constrains a drug 124 to be administered within the syringe body 116. Whilst the syringe illustrated is of hypodermic type, this need not necessarily be so. Transcutaneous or ballistic dermal and subcutaneous syringes may also be used with the injection device of the present invention. As illustrated, the housing 112 includes a return spring 126 that biases the syringe 114 from an extended position in which the needle 118 extends from an aperture 128 in the housing 112 to a retracted position in which the discharge nozzle 118 is contained within the housing 112. The return spring 126 acts on the syringe 114 via a syringe carrier 127.

At the other end of the housing is an actuator, which here takes the form of a compression drive spring 130. Drive from the drive spring 130 is transmitted via a multi-component drive to the syringe 114 to advance it from its retracted position to its extended position and discharge its contents through the needle 118. The drive accomplishes this task by acting directly on the drug 124 and the syringe 114. Hydrostatic forces acting through the drug 124 and, to a lesser extent, static friction between the bung 122 and the syringe body 116 initially ensure that they advance together, until the return spring 126 bottoms out or the syringe body 116 meets some other obstruction (not shown) that retards its motion.

The multi-component drive between the drive spring 130 and the syringe 114 consists of three principal components. A drive sleeve 131 takes drive from the drive spring 130 and transmits it to flexible latch arms 133 on a first drive element 132. This in turn transmits drive via flexible latch arms 135 to a second drive element, the drive element 134 already mentioned.

The first drive element 132 includes a hollow stem 140, the inner cavity of which forms a collection chamber 142 in communication with a vent 144 that extends from the collection chamber through the end of the stem 140. The second drive element 134 includes a blind bore 146 that is open at one end to receive the stem 140 and closed at the other. As can be seen, the bore 146 and the stem 140 defining a fluid reservoir 148, within which a damping fluid is contained.

A trigger (not shown) is provided that, when operated, serves to decouple the drive sleeve 131 from the housing 112, allowing it to move relative to the housing 112 under the influence of the drive spring 130. The operation of the device is then as follows.

Initially, the drive spring 130 moves the drive sleeve 131, the drive sleeve 131 moves the first drive element 32 and the first drive element 132 moves the second drive element 134, in each case by acting through the flexible latch arms 133, 135. The second drive element 134 moves and, by virtue of static friction and hydrostatic forces acting through the drug 124 to be administered, moves the syringe body 116 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug 124 to be administered are not sufficient to resist the full drive force developed by the drive spring 130, at this point the second drive element 134 begins to move within the syringe body 116 and the drug 124 begins to be discharged. Dynamic friction between the second drive element 134 and the syringe body 116 and hydrostatic forces acting through the drug 124 to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 135 linking the first and second drive elements 132, 134 reach a constriction 137 within the housing 112. The constriction 137 moves the flexible latch arms 135 inwards from the position shown to a position at which they no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir 148 defined between the end of the first drive element 132 and the blind bore 146 in the second drive element 134, the volume of the reservoir 146 will tend to decrease as the first drive element 132 moves relative to the second drive element 134 when the former is acted upon by the drive spring 130. As the reservoir 148 collapses, damping fluid is forced through the vent 144 into the collection chamber 142. Thus, once the flexible latch arms 135 have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir 148 of fluid is exhausted, the flexible latch arms 133 linking the drive sleeve 131 with the first drive element 132 reach another constriction 139 within the housing 112. The constriction 139 moves the flexible latch arms 133 inwards from the position shown to a position at which they no longer couple the drive sleeve 131 to the first drive element 132, aided by the bevelled surfaces on the constriction 139. Once this happens, the drive sleeve 131 acts no longer on the first drive element 132, allowing them to move relative each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. As can be seen from fig. 3, the end of the syringe is sealed with a boot 123. The central boss 121 of the cap that fits within the sleeve 119 when the cap 111 is installed on the housing 112, is hollow at the end and the lip 125 of the hollow end is bevelled on its leading edge 157, but not its trailing edge. Thus, as the cap 111 is installed, the leading edge 157 of the lip 125 rides over a shoulder 159 on the boot 123. However, as the cap 111 is removed, the trailing edge of the lip 125 will not ride over the shoulder 159, which means that the boot 123 is pulled off the syringe 114 as the cap 111 is removed.

Meanwhile, as can best be seen in Figs. 4a, 4b and 5, the syringe carrier 127, with respect to which the syringe 114 cannot move, is prevented from movement by a resilient latch member 161 that is located within the housing 112 and is biased into a position in which it engages a locking surface 163 of a syringe carrier 127. As shown in Fig. 4a, before engaging the locking surface 163, the latch member 161 also extends through a latch opening 165 in the sleeve 119, the end of which projects from the exit aperture 128. The latch member 161 includes a ramped surface 167 against which an edge 171 of the latch opening 165 acts in the manner of a cam acting on a cam follower. Thus, movement of the sleeve 119 in a direction into the housing 112, or in other words depression of the flange 119 towards rim 128a, brings the edge 171 of the latch opening 165 into contact with the ramped surface 167 of the latch member 161 and further depression, as shown in Fig. 4b, causes the latch member 161 to move outwards and thus to disengage from the locking surface 163. The sleeve 119 may be depressed by bringing the flange 119 into contact with the skin at an injection site and bringing the injection device 110 towards the skin. Once the latch member 161 has disengaged from the locking surface 163, the syringe carrier 127 is free to move as required under the influence of the actuator and drive.

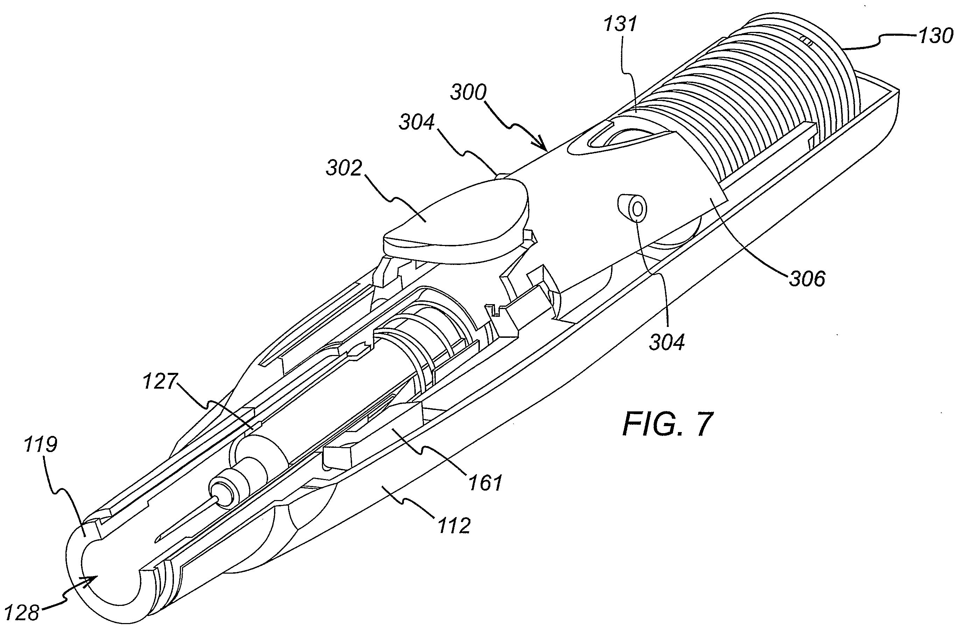

Figs. 6 and 7 show how the device may be further modified. Although figs. 6 and 7 differ from Figs. 4a, 4b and 5 in some details, the principles now discussed are applicable to the device shown in Figs. 4a, 4b and 5. As can be seen, the device includes a trigger 300 having a button 302 at one end and a pair of lugs 304 that cooperate with pins (not shown) on the inside of the housing 112 to allow the trigger to pivot about an axis through the two lugs 304. The main body portion of the trigger 300, to which both the button 302 and the lugs 304 are affixed, forms a locking member 306. In the position shown, the end of the locking member 306 remote from the button 302 engages the end of the drive sleeve 131, against which the drive spring 130 acts and which in turn acts upon the multi-component drive previously discussed. This prevents the drive sleeve 131 from moving under the influence of the drive spring 130. When the button 302 is depressed, the trigger 300 pivots about the lugs 304, which lifts the end of the locking member 306 from its engagement with the drive sleeve 131, now allowing the drive sleeve 131 to move under the influence of the drive spring 130.

Fig. 7 shows the exit aperture 128 in the end of the housing 112, from which the end of the sleeve 119 can again be seen to emerge. As is shown in fig. 6, the sleeve 119 is coupled to a button lock 310 which moves together with the sleeve 119. The trigger includes a stop pin 312 and the button lock 310 includes an stop aperture 314 which, as shown in fig. 6, are out of register. They can, however, be brought into register by inward movement of the sleeve 119, which results in a corresponding movement of the button lock 310. Whilst the stop pin 312 and the stop aperture 314 are out of register, the button 302 may not be depressed; once they are in register, it may. The trigger 300 also includes a flexible, barbed latching projection 316 and the button lock 310 also includes a latching surface 318 with which the latching projection 316 engages when the button is depressed. Once the latching projection 316 has latched with the latching surface 318, the trigger 300 is permanently retained with the button 302 in its depressed position.

Thus, movement of the sleeve 119 in a direction into the housing 112, or in other words depression of the projecting end of the sleeve, brings the stop pin 312 into register with the stop aperture 314, allowing the trigger button 302 to be depressed, whereupon it is retained in its depressed position by the latching projection 316 and the latching surface 318. The sleeve 119 may be depressed by bringing the end of the injection device into contact with the skin at an injection site which, apart from anything else, ensures it is properly positioned before the injection cycle begins.

The use of the sleeve 119 both the release and lock the trigger 300 and to allow the syringe carrier 127 to move, together with a boot-removing cap 111 that prevents the sleeve 119 from being depressed results in an integrated injection device of elegant design.

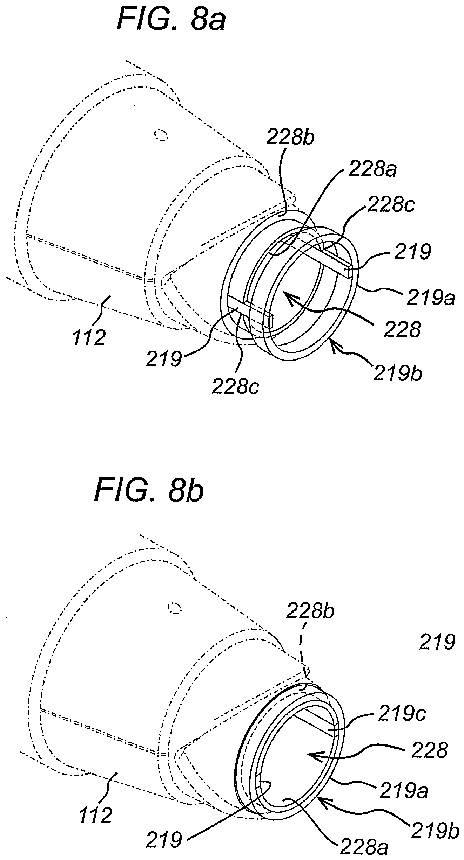

Fig. 8 shows and alternative embodiment of the end of the injection device 110. in exactly the same ways as discussed in connection with Fig. 1, the end of the housing 112 has an exit aperture 228 formed by rim 228a. Arms 219 which form part of the locking mechanism in exactly the same way as the sleeve 119 in Figs. 1 to 5, emerge from the exit aperture 228. Each arm 219 is connected to a cylindrical end section 219a having an aperture. Each arm 219 is connected on the inside of the aperture. In a similar way to the flange 119a, the cylindrical end section 219a has a contact surface 219b which can contact tissue when pressed against it. The arms 219 sit and slide in slots 228c which extend through the end of the rim 228a. A shelf 228b on the housing extends around the circumference of the rim 228a and is adapted to receive the cylindrical end section 219a and prevent rearwards movement.

The cylindrical end section 219a can slide from a locked position in which the cylindrical end section 219a is spaced from the shelf 228b, to an unlocked position in which the cylindrical end section 219a has been pushed into a position in which it sits adjacent, in contacting juxtaposition, to the shelf 228b around the outside of the rim 228a. In all other aspects, the injection device 110 and locking mechanism operates in the same way as the sleeve 119 explained in connection with Figs. 4a, 4b and 5 above.

It will of course be understood that the present invention has been described above purely by way of example and modifications of detail can be made within the scope of the invention, as defined by the appended claims.

## Claims

1. An injection device (110) comprising:
a housing (112) adapted to receive a syringe (114) having a discharge nozzle (118), the syringe (114) being moveable in the housing (112) on actuation of the injection device (110) along a longitudinal axis from a retracted position in which the discharge nozzle (118) is contained within the housing (112) and an extended position in which the discharge nozzle (118) of the syringe (114) extends from the housing (112) through an exit aperture (128);
an actuator (130);
a drive adapted to be acted upon by the actuator (130) and in turn act upon the syringe (114) to advance it from its retracted position to its extended position and discharge its contents through the discharge nozzle (128);
a locking mechanism moveable from an engaged position in a direction into the housing (112) at the exit aperture (128) into a disengaged position,
wherein the locking mechanism is adapted to prevent actuation of the device when it is in its engaged position and permit actuation of the device when it is in its disengaged position,
wherein the exit aperture (128) is defined by a rim (128a) located on an edge of the housing (112), and
wherein the locking mechanism comprises a contact surface which is adapted to extend over or around at least a part of the rim (128a).
**characterised in that** the locking mechanism further comprises at least one arm (219) extending from the contact surface into the housing (112).

2. The injection device (110) of claim 1, wherein the at least one arm (219) extends into the housing (112) through the exit aperture (128).

3. The injection device (110) of claim 2, wherein the rim (128a) of the exit aperture (128) is elliptical or circular and the at least one arm (219) comprises an elliptical or circular cross-section shaped and positioned in such a way that it first within an inner surface of the exit aperture (128).

4. The injection device (110) of any one of claims 1 to 3, wherein the rim (128a) comprises an opening through which the at least one arm (219) extends into the housing (112).

5. The injection device (110) of any preceding claim, wherein the locking mechanism comprises a pair of arms (219).

6. The injection device (110) of any one of the preceding claims, wherein the contact surface (219b) is formed on a first side of a hoop (219a).

7. The injection device (110) of any one of the preceding claims, wherein a second side of the hoop (219a) opposite the first side faces the rim (128a) of the exit aperture (128).

8. The injection device (110) of any one of the preceding claims, wherein the second side moves towards the rim (128a) when the locking mechanism is moved from its engaged position to its disengaged position.

9. The injection device (110) of any one of the preceding claims, wherein the release mechanism is arranged on the injection device (110) such that an inner radius of the hoop (219a) surrounds the rim (128a) when the release mechanism is in its disengaged position.

10. The injection device (110) of any one of the preceding claims, further comprising:
a syringe carrier (127) for carrying the syringe (114) as it is advanced and restraining its advancement beyond its extended position, wherein the syringe carrier (127) is adapted to support the syringe (114);
a latch member (161) adapted to prevent, in an engaged position of the locking mechanism, movement of the syringe carrier (127) relative to the housing (112) and further adapted to permit, in a disengaged position of the locking mechanism, the syringe carrier (127) moving relative to the housing (112).

11. The injection device (110) of claim 10, wherein the locking mechanism comprises a primary member movable between the engaged position and the disengaged position.

12. The injection device (110) of claim 10 when dependent on any one of claims 1 to 5, wherein the primary member is the at least one arm (219) connected to the contact surface (219b).

13. The injection device (110) of claim 11 or 12, wherein the primary member includes a latch opening (165) through which the latch member (161) projects before it engages a locking surface (163) on the syringe carrier (127), the primary member acting as a cam and the latch member (161) as a cam follower, so that movement of the primary member from its engaged position to its disengaged position causes the latch member (161) to disengage from the locking surface (163).

14. The injection device (110) of claim 13, wherein the latch member (161) includes a ramped surface (167) against which a surface of the primary member acts to disengage it from the locking surface (163).

15. The injection device (110) of claim 14, wherein the latch member (161) is provided on the housing (112).

16. The injection device (110) of any one of the preceding claims, further comprising a release mechanism moveable between an unactuated position and an actuated position,
wherein the release mechanism is adapted to prevent the actuator (130) acting on the drive when in its unactuated position and permits the actuator (130) to act on the drive when in its actuated position.

17. The injection device (110) of claim 16, wherein the locking mechanism further comprises an interlock member movable between a locking position when the locking mechanism is in its engaged position, at which it prevents movement of the release mechanism from its unactuated position to its actuated position, and a releasing position when the release mechanism is in its disengaged position, at which it allows movement of the release mechanism from its unactuated position to its actuated position.

## Patentansprüche

1. Injektionsvorrichtung (110), umfassend:
ein Gehäuse (112), das zur Aufnahme einer Spritze (114) geeignet ist, die eine Austragdüse (118) hat, wobei die Spritze (114) bei Betätigung der Injektionsvorrichtung (110) im Gehäuse (112) entlang einer Längsachse aus einer eingezogenen Position, in der die Austragdüse (118) im Gehäuse (112) enthalten ist, und einer ausgefahrenen Position, in der sich die Austragdüse (118) der Spritze (114) durch eine Austrittsöffnung (128) aus dem Gehäuse (112) erstreckt, beweglich ist,
einen Aktuator (130),
einen Antrieb, der geeignet ist, dass der Aktuator (130) auf ihn einwirkt, und seinerseits auf die Spritze (114) einwirkt, um sie aus ihrer eingezogenen Position in ihre ausgefahrene Position vorzuschieben und ihren Inhalt durch die Austragdüse (128) auszutragen,
einen Sperrmechanismus, der aus einer eingerückten Position in einer Richtung in das Gehäuse (112) an der Austrittsöffnung (128) in eine ausgerückte Position beweglich ist,
wobei der Sperrmechanismus geeignet ist, eine Betätigung der Vorrichtung zu verhindern, wenn er in seiner eingerückten Position ist, und eine Betätigung der Vorrichtung zu gestatten, wenn er in seiner ausgerückten Position ist,
wobei die Austrittsöffnung (128) durch einen an einer Kante des Gehäuses (112) angeordneten Rand (128a) definiert ist und
wobei der Sperrmechanismus eine Kontaktfläche umfasst, die geeignet ist, sich über oder um mindestens einen Teil des Rands (128a) herum zu erstrecken,
**dadurch gekennzeichnet, dass** der Sperrmechanismus ferner mindestens einen Arm (219) umfasst, der sich von der Kontaktfläche in das Gehäuse (112) erstreckt.

2. Injektionsvorrichtung (110) nach Anspruch 1, wobei sich der mindestens eine Arm (219) durch die Austrittsöffnung (128) in das Gehäuse (112) erstreckt.

3. Injektionsvorrichtung (110) nach Anspruch 2, wobei der Rand (128a) der Austrittsöffnung (128) elliptisch oder kreisförmig ist und der mindestens eine Arm (219) einen elliptischen oder kreisförmigen Querschnitt hat, der so gestaltet und positioniert ist, dass er in eine Innenfläche der Austrittsöffnung (128) passt.

4. Injektionsvorrichtung (110) nach einem der Ansprüche 1 bis 3, wobei der Rand (128a) eine Öffnung umfasst, durch die sich der mindestens eine Arm (219) in das Gehäuse (112) erstreckt.

5. Injektionsvorrichtung (110) nach einem der vorhergehenden Ansprüche, wobei der Sperrmechanismus ein Paar Arme (219) umfasst.

6. Injektionsvorrichtung (110) nach einem der vorhergehenden Ansprüche, wobei die Kontaktfläche (219b) an einer ersten Seite eines Reifens (219a) ausgebildet ist.

7. Injektionsvorrichtung (110) nach einem der vorhergehenden Ansprüche, wobei eine zweite Seite des Reifens (219a) gegenüber der ersten Seite dem Rand (128a) der Austrittsöffnung (128) zugewandt ist.

8. Injektionsvorrichtung (110) nach einem der vorhergehenden Ansprüche, wobei sich die zweite Seite zum Rand (128a) hin bewegt, wenn der Sperrmechanismus aus seiner eingerückten Position in seine ausgerückte Position bewegt wird.

9. Injektionsvorrichtung (110) nach einem der vorhergehenden Ansprüche, wobei der Freigabemechanismus so an der Injektionsvorrichtung (110) angeordnet ist, dass ein innerer Radius des Reifens (219a) den Rand (128a) umgibt, wenn der Freigabemechanismus in seiner ausgerückten Position ist.

10. Injektionsvorrichtung (110) nach einem der vorhergehenden Ansprüche, ferner umfassend:
einen Spritzenträger (127), um die Spritze (114) zu tragen, während sie vorgeschoben wird, und um ihren Vorschub über ihre ausgefahrene Position hinaus zu hemmen, wobei der Spritzenträger (127) zum Stützen der Spritze (114) geeignet ist,
ein Riegelglied (161), das geeignet ist, in einer eingerückten Position des Sperrmechanismus eine Bewegung des Spritzenträgers (127) bezüglich des Gehäuses (112) zu verhindern, und ferner geeignet ist, in einer ausgerückten Position des Sperrmechanismus zu gestatten, dass sich der Spritzenträger (127) bezüglich des Gehäuses (112) bewegt.

11. Injektionsvorrichtung (110) nach Anspruch 10, wobei der Sperrmechanismus ein primäres Glied umfasst, das zwischen der eingerückten Position und der ausgerückten Position beweglich ist.

12. Injektionsvorrichtung (110) nach Anspruch 10, wenn er von einem der Ansprüche 1 bis 5 abhängig ist, wobei das primäre Glied der mindestens eine mit der Kontaktfläche (219b) verbundene Arm (219) ist.

13. Injektionsvorrichtung (110) nach Anspruch 11 oder 12, wobei das primäre Glied eine Riegelöffnung (165) aufweist, durch welche das Riegelglied (161) vorragt, bevor es eine Sperrfläche (163) am Spritzenträger (127) in Eingriff nimmt, wobei das primäre Glied als eine Nocke und das Riegelglied (161) als ein Mitnehmer wirken, so dass durch eine Bewegung des primären Glieds aus seiner eingerückten Position in seine ausgerückte Position veranlasst wird, dass das Riegelglied (161) aus der Sperrfläche (163) ausrückt.

14. Injektionsvorrichtung (110) nach Anspruch 13, wobei das Riegelglied (161) eine Rampenfläche (167) aufweist, gegen die eine Fläche des primären Glieds wirkt, um es von der Sperrfläche (163) auszurücken.

15. Injektionsvorrichtung (110) nach Anspruch 14, wobei das Riegelglied (161) am Gehäuse (112) vorgesehen ist.

16. Injektionsvorrichtung (110) nach einem der vorhergehenden Ansprüche, ferner umfassend einen Freigabemechanismus, der zwischen einer unbetätigten Position und einer betätigten Position beweglich ist,
wobei der Freigabemechanismus geeignet ist, zu verhindern, dass der Aktuator (130) auf den Antrieb wirkt, wenn er in seiner unbetätigten Position ist, und gestattet, dass der Aktuator (130) auf den Antrieb wirkt, wenn er in seiner betätigten Position ist.

17. Injektionsvorrichtung (110) nach Anspruch 16, wobei der Sperrmechanismus ferner ein Verriegelungsglied umfasst, das zwischen einer Sperrposition, wenn der Sperrmechanismus in seiner eingerückten Position ist, in der es eine Bewegung des Freigabemechanismus aus seiner unbetätigten Position in seine betätigte Position verhindert, und einer Freigabeposition beweglich ist, wenn der Freigabemechanismus in seiner ausgerückten Position ist, in der es eine Bewegung des Freigabemechanismus aus seiner unbetätigten Position in seine betätigte Position gestattet.

## Revendications

1. Dispositif d'injection (110), comprenant :
un boîtier (112) prévu pour recevoir une seringue (114) ayant une buse de décharge (118), la seringue (114) pouvant être déplacée dans le boîtier (112) lors de l'actionnement du dispositif d'injection (110) le long d'un axe longitudinal, depuis une position rentrée dans laquelle la buse de décharge (118) est contenue à l'intérieur du boîtier (112) jusqu'à une position étendue, dans laquelle la buse de décharge (118) de la seringue (114) s'étend depuis le boîtier (112) à travers une ouverture de sortie (128) ;
un actionneur (130) ;
un entraînement sur lequel l'actionneur (130) peut agir et qui à son tour agit sur la seringue (114) pour la faire avancer de sa position rentrée jusqu'à sa position étendue et décharger son contenu à travers la buse de décharge (128) ;
un mécanisme de verrouillage pouvant être déplacé d'une position engagée en direction de l'intérieur du boîtier (112) au niveau de l'ouverture de sortie (128) jusque dans une position désengagée,
le mécanisme de verrouillage étant prévu pour empêcher l'actionnement du dispositif lorsqu'il est dans sa position engagée et pour permettre l'actionnement du dispositif lorsqu'il est dans sa position désengagée,
l'ouverture de sortie (128) étant définie par un rebord (128a) situé sur un bord du boîtier (112), et
le mécanisme de verrouillage comprenant une surface de contact qui est prévue pour s'étendre par-dessus ou autour d'au moins une partie du rebord (128a),
**caractérisé en ce que** le mécanisme de verrouillage comprend en outre au moins un bras (219) s'étendant depuis la surface de contact jusque dans le boîtier (112).

2. Dispositif d'injection (110) selon la revendication 1, dans lequel l'au moins un bras (219) s'étend dans le boîtier (112) à travers l'ouverture de sortie (128).

3. Dispositif d'injection (110) selon la revendication 2, dans lequel le rebord (128a) de l'ouverture de sortie (128) est elliptique ou circulaire et l'au moins un bras (219) comprend une section transversale elliptique ou circulaire formée et positionnée de telle sorte qu'il s'ajuste à l'intérieur d'une surface interne de l'ouverture de sortie (128).

4. Dispositif d'injection (110) selon l'une quelconque des revendications 1 à 3, dans lequel le rebord (128a) comprend une ouverture à travers laquelle l'au moins un bras (219) s'étend jusque dans le boîtier (112).

5. Dispositif d'injection (110) selon l'une quelconque des revendications précédentes, dans lequel le mécanisme de verrouillage comprend une paire de bras (219).

6. Dispositif d'injection (110) selon l'une quelconque des revendications précédentes, dans lequel la surface de contact (219b) est formée sur un premier côté d'une boucle (219a).

7. Dispositif d'injection (110) selon l'une quelconque des revendications précédentes, dans lequel un deuxième côté de la boucle (219a) opposé au premier côté fait face au rebord (128a) de l'ouverture de sortie (128).

8. Dispositif d'injection (110) selon l'une quelconque des revendications précédentes, dans lequel le deuxième côté se déplace vers le rebord (128a) lorsque le mécanisme de verrouillage est déplacé de sa position engagée à sa position désengagée.

9. Dispositif d'injection (110) selon l'une quelconque des revendications précédentes, dans lequel le mécanisme de libération est disposé sur le dispositif d'injection (110) de telle sorte qu'un rayon interne de la boucle (219a) entoure le rebord (128a) lorsque le mécanisme de libération est dans sa position désengagée.

10. Dispositif d'injection (110) selon l'une quelconque des revendications précédentes, comprenant en outre :
un support de seringue (127) destiné à supporter la seringue (114) au fur et à mesure qu'elle est avancée et à limiter sa progression au-delà de sa position étendue, le support de seringue (127) étant prévu pour supporter la seringue (114) ;
un organe de loquet (161) prévu pour empêcher, dans une position engagée du mécanisme de verrouillage, un mouvement du support de seringue (127) par rapport au boîtier (112) et en outre prévu pour permettre, dans une position désengagée du mécanisme de verrouillage, le déplacement du support de seringue (127) par rapport au boîtier (112) .

11. Dispositif d'injection (110) selon la revendication 10, dans lequel le mécanisme de verrouillage comprend un organe primaire pouvant être déplacé entre la position engagée et la position désengagée.

12. Dispositif d'injection (110) selon la revendication 10 lorsqu'elle dépend de l'une quelconque des revendications 1 à 5, dans lequel l'organe primaire est l'au moins un bras (219) connecté à la surface de contact (219b).

13. Dispositif d'injection (110) selon la revendication 11 ou 12, dans lequel l'organe primaire comporte une ouverture de loquet (165) à travers laquelle l'organe de loquet (161) fait saillie avant de s'engager avec une surface de verrouillage (163) sur le support de seringue (127), l'organe primaire agissant en tant que came et l'organe de loquet (161) agissant en tant que suiveur de came de telle sorte que le mouvement de l'organe primaire de sa position engagée à sa position désengagée entraîne le désengagement de l'organe de loquet (161) de la surface de verrouillage (163).

14. Dispositif d'injection (110) selon la revendication 13, dans lequel l'organe de loquet (161) comporte une surface en rampe (167) contre laquelle une surface de l'organe primaire agit de manière à le désengager de la surface de verrouillage (163).

15. Dispositif d'injection (110) selon la revendication 14, dans lequel l'organe de loquet (161) est prévu sur le boîtier (112).

16. Dispositif d'injection (110) selon l'une quelconque des revendications précédentes, comprenant en outre un mécanisme de libération pouvant être déplacé entre une position non actionnée et une position actionnée,
le mécanisme de libération étant prévu pour empêcher que l'actionneur (130) n'agisse sur l'entraînement lorsqu'il est dans sa position non actionnée et permettant à l'actionneur (130) d'agir sur l'entraînement lorsqu'il est dans sa position actionnée.

17. Dispositif d'injection (110) selon la revendication 16, dans lequel le mécanisme de verrouillage comprend en outre un organe d'emboîtement pouvant être déplacé entre une position de verrouillage lorsque le mécanisme de verrouillage est dans sa position engagée, dans laquelle il empêche le mouvement du mécanisme de libération de sa position non actionnée à sa position actionnée, et une position de libération lorsque le mécanisme de libération est dans sa position désengagée, dans laquelle il permet le mouvement du mécanisme de libération de sa position non actionnée à sa position actionnée.
